# EUROPEAN PATENT SPECIFICATION

(11) **EP 3 692 846 B1**
(45) Date of publication and mention of the grant of the patent: **20.04.2022**
(21) Application number: 20166260.8
(22) Date of filing: 08.05.2017
(51) Int. Cl.: A24F 47/00, H05B 6/00, H05B 3/00

(54) **AEROSOL-GENERATING DEVICE COMPRISING A SENSOR**
AEROSOLERZEUGUNGSARTIKEL MIT EINEM FLÜSSIGKEITSINDIKATOR
ARTICLE DE GÉNÉRATION D'AÉROSOL AYANT UN INDICATEUR DE LIQUIDE

(30) Priority: 27.05.2016 EP 16171790
(43) Date of publication of application: 12.08.2020
(62) Divisional of application: 17720842.8
(73) Proprietor: Philip Morris Products S.A., 2000 Neuchâtel (CH)
(72) Inventor: BILAT, Stephane, 2015 Areuse (CH); COLOTTE, Guillaume, 1003 Lausanne (CH)
(74) Representative: Dowling, Ian

(56) References cited:
- EP-A1- 2 201 850
- WO-A1-2015/042412
- WO-A1-2015/082560
- WO-A1-2015/150068
- WO-A1-2016/005602
- WO-A2-2013/060743
- DE-U1-202004 017 418
- US-A1- 2014 060 554

## Description

The present description relates to an aerosol-generating article comprising a hydrochromic material, an aerosol-generating system comprising the aerosol-generating article, and an aerosol-generating device comprising an electronic photosensor. The invention is set out in the appended claims.

WO 2015/082560 A1 concerns the identification of aerosol-generating articles based on optical recognition of a taggant provided on the articles. WO 2015/082560 A1 describes a method of controlling an aerosol-generating system based on detecting whether a recognised taggant is present, and either preventing activation of a device if no recognised taggant is detected, or activating the device is a recognised taggant is detected. WO 2015/082560 A1 also describes an aerosol-generating system configured to carry out the method, the system comprising a detector for detecting the spectroscopic signature of a taggant of an aerosol-generating article. In an embodiment, an aerosol-generating article comprises a container, wherein a taggant is incorporated into the material forming the container. An aerosol-generating device comprises a detector comprising a light source and a light sensor for determining the spectroscopic signature of the taggant to identify the aerosol-generating article. The detector enables the aerosol-generating device to determine the type of aerosol-generating article inserted into the device. Control circuitry supplies power from a power supply to a heater according to the determined type of article. If the article is not recognised by the detector, the control circuitry may prevent the supply of power to the heater to prevent the use of unauthorised articles with the device.

One type of aerosol-generating system is an electronic cigarette. Electronic cigarettes typically use a liquid aerosol-forming substrate which is vaporised to form an aerosol. An electronic cigarette typically comprises a power supply, a liquid storage portion for holding a supply of the liquid aerosol-forming substrate and an atomiser.

The liquid aerosol-forming substrate becomes exhausted in use and so needs to be replenished. The most common way to supply refills of liquid aerosol-forming substrate is in a cartomiser type cartridge. The cartomiser may be regarded as a consumable aerosol-generating article, and the reusable part of the electronic cigarette may be regarded as an aerosol-generating device. A cartomiser may comprises both a supply of liquid substrate and the atomiser, usually in the form of an electrically operated resistance heater wound around a capillary material soaked in the aerosol-forming substrate. Replacing a cartomiser as a single consumable unit has the benefit of being convenient for the user and avoids the need for the user to have to clean or otherwise maintain the atomiser. However, it may be difficult for a user to determine when the liquid aerosol-forming substrate in a cartomiser has been exhausted. Therefore, it may be difficult for a user to determine when to replace a consumable part of an aerosol-generating system, such as the cartomiser of an electronic cigarette.

It would be desirable to provide an aerosol-generating article comprising a liquid aerosol-forming substrate and an indicator to indicate when the liquid aerosol-forming substrate has been exhausted

According to a first aspect of the present description there is provided an aerosol-generating article comprising a liquid storage portion containing a liquid aerosol-forming substrate, and a hydrochromic material provided on the liquid storage portion. The hydrochromic material has a first colour when in contact with the liquid aerosol-forming substrate and a second colour in the absence of the liquid aerosol-forming substrate.

As used herein, the term "aerosol-forming substrate" is used to describe a substrate capable of releasing volatile compounds, which can form an aerosol. The aerosols generated from aerosol-forming substrates of aerosol-generating articles according to the invention may be visible or invisible and may include vapours (for example, fine particles of substances, which are in a gaseous state, that are ordinarily liquid or solid at room temperature) as well as gases and liquid droplets of condensed vapours.

Aerosol-generating articles according to the present invention comprise a hvdrochromic material configured to exhibit a change in colour when the liquid aerosol-forming substrate has been exhausted. Advantageously, the hydrochromic material provides a simple and cost-effective indication of exhaustion of the liquid aerosol-forming substrate.

Advantageously, aerosol-generating articles according to the present invention may minimise the risk of a user discarding the aerosol-generating article before all of the liquid aerosol-forming substrate has been consumed.

Advantageously, aerosol-generating articles according to the present invention may minimise the risk of a user continuing to use the aerosol-generating article after all of the liquid aerosol-forming substrate has been consumed. In embodiments in which the liquid aerosol-forming substrate is heated to generate an aerosol, minimising the risk of continued heating of a dry aerosol-generating article may be desirable to prevent the release of one or more undesirable substances from the aerosol-generating article.

The hydrochromic material may be provided as a coating on at least a portion of the liquid storage portion. The hydrochromic material may comprise at least one of an ink and a paint. Hydrochromic inks and paints may be particularly advantageous in embodiments in which the hydrochromic material is provided as a coating.

One of the first colour and the second colour may be a condition in which the hydrochromic material is substantially colourless. The term "colourless" is used herein to refer to a material that transmits light substantially equally across the visible portion of the electromagnetic spectrum.

At least one of the first colour and the second colour may be translucent or transparent. Translucent and transparent materials transmit at least 50 percent of incident light for at least one wavelength in the visible portion of the electromagnetic spectrum. The term "translucent" is used herein to refer to a material that transmits light with scattering. The term "transparent" is used herein to refer to a material that transmits light substantially without scattering.

Translucent and transparent materials may be substantially colourless.

Translucent and transparent materials may transmit some wavelengths of light more than others so that the translucent or transparent material is not colourless.

At least one of the first colour and the second colour may be opaque. The term "opaque" is used herein to refer to a material that reflects or absorbs more than 50 percent of incident light for all wavelengths of the visible portion of the electromagnetic spectrum. An opaque material that absorbs all wavelengths exhibits a black colour. An opaque material that reflects all wavelengths exhibits a colour corresponding to the colour of the incident light. An opaque material that absorbs some wavelengths and reflects the remaining wavelengths exhibits a colour corresponding to the combination of the reflected wavelengths of the incident light.

The hydrochromic material may comprise one or more pigments or dyes to provide a desired first colour, second colour, or both. The hydrochromic material may comprise one or more inorganic pigments or dyes. The hydrochromic material may comprise one or more organic pigments or dyes. Suitable pigments and dyes include azo dyes, anthraquinone dyes, xanthene dyes, azine dyes, and combinations thereof.

The hydrochromic material may gradually change from the first colour to the second colour as the liquid aerosol-forming substrate is consumed during use of the aerosol-generating article. In this way, the hydrochromic material may provide an indication of the amount of liquid aerosol-forming substrate remaining in the liquid storage portion. For example, in embodiments in which the first colour is translucent or transparent and the second colour is an opaque colour, the hydrochromic material may exhibit a gradual increase in opacity as the liquid aerosol-forming substrate is consumed.

The liquid aerosol-forming substrate may comprise water. Preferably, the hydrochromic material changes colour in response to the presence or absence of water.

The hydrochromic material may comprise at least one of a finely particulate silicic acid, a barite powder, precipitated barium sulfate, barium carbonate, precipitated calcium carbonate, gypsum, clay, talc, alumna white, basic magnesium carbonate, and combinations thereof.

The aerosol-generating article may comprise a base layer, wherein the liquid storage portion comprises a porous substrate material positioned on the base layer and the liquid aerosol-forming substrate sorbed into the porous substrate material. Preferably, the hydrochromic material is provided on an outer surface of the porous substrate material. That is, the hydrochromic material is preferably provided on a surface of the porous substrate material that may be visible to a user.

The aerosol-generating article may comprise a single porous substrate material and a single liquid aerosol-forming substrate sorbed into the porous substrate material.

The aerosol-generating article may comprise a plurality of discrete segments of porous substrate material positioned on the base layer, wherein the liquid aerosol-forming substrate comprises a liquid aerosol-forming substrate sorbed into each segment of porous substrate material. The liquid aerosol-forming substrates may be substantially the same. At least one of the liquid aerosol-forming substrates may be different from the remaining liquid aerosol-forming substrates.

Preferably, each porous substrate material has a density of between about 0.1 grams/cubic centimetre and about 0.3 grams/cubic centimetre.

Preferably, each porous substrate material has a porosity of between about 15 percent and about 55 percent.

Each porous substrate material may comprise one or more of glass, cellulose, ceramic, stainless steel, aluminium, polyethylene (PE), polypropylene, polyethylene terephthalate (PET), poly(cyclohexanedimethylene terephthalate) (PCT), polybutylene terephthalate (PBT), polytetrafluoroethylene (PTFE), expanded polytetrafluoroethylene (ePTFE), and BAREX^{®}.

Preferably, each porous carrier material is chemically inert with respect to the liquid aerosol-forming substrate sorbed into the porous carrier material.

The base layer and each porous carrier material may be in contact with each other at a substantially planar contact surface. Providing each porous carrier material on a substantially planar portion of the base layer may simplify the manufacture of the aerosol-generating article.

As used herein, the term "substantially planar", means arranged substantially along a single plane.

The aerosol-generating article may comprise a cover layer sealed to the base layer so that each porous substrate material is sealed between the base layer and the cover layer. Preferably, the cover layer is sealed to the base layer around a periphery of the base layer.

The cover layer may be configured to be removable from the base layer prior to use of the aerosol-generating article.

The cover layer may be configured to remain on the base layer during use of the aerosol-generating article. For example, the cover layer may be pierced prior to use of the aerosol-generating article. In embodiments in which the cover layer is configured to remain on the base layer during use of the aerosol-generating article, at least a portion of the cover layer overlying the hydrochromic material may be translucent or transparent.

The base layer may have any suitable cross-sectional shape. Preferably, the base layer has a non-circular cross-sectional shape. The base layer may have a substantially rectangular cross-sectional shape. The base layer may have an elongate, substantially rectangular, parallelepiped shape. The base layer may be substantially flat. The base layer may be substantially planar. A substantially planar base layer may be particularly suited to aerosol-generating articles comprising at least one solid aerosol-forming substrate.

The base layer may comprises a polymeric foil.

The liquid aerosol-forming substrate may comprise a liquid nicotine source sorbed into a porous substrate material.

Preferably, the liquid nicotine source comprises one or more of nicotine, nicotine base, a nicotine salt, such as nicotine-HCI, nicotine-bitartrate, or nicotine-ditartrate, or a nicotine derivative.

The nicotine source may comprise natural nicotine or synthetic nicotine.

The nicotine source may comprise pure nicotine, a solution of nicotine in an aqueous or non-aqueous solvent or a liquid tobacco extract.

The nicotine source may comprise an electrolyte forming compound. The electrolyte forming compound may be selected from the group consisting of alkali metal hydroxides, alkali metal oxides, alkali metal salts, alkaline earth metal oxides, alkaline earth metal hydroxides and combinations thereof.

The nicotine source may comprise an electrolyte forming compound selected from the group consisting of potassium hydroxide, sodium hydroxide, lithium oxide, barium oxide, potassium chloride, sodium chloride, sodium carbonate, sodium citrate, ammonium sulfate and combinations thereof.

The nicotine source may comprise an aqueous solution of nicotine, nicotine base, a nicotine salt or a nicotine derivative and an electrolyte forming compound.

The nicotine source may comprise other components including, but not limited to, natural flavours, artificial flavours and antioxidants.

The liquid aerosol-forming substrate may comprise a first liquid aerosol-forming substrate comprising the nicotine source sorbed into a first porous substrate material, and a second liquid aerosol-forming substrate comprising an acid source sorbed into a second porous substrate material. During use, volatile compounds from the nicotine source and the acid source may react in the gas phase to form an aerosol comprising nicotine salt particles.

The acid source may comprise an organic acid or an inorganic acid. Preferably, the acid source comprises an organic acid, more preferably a carboxylic acid, most preferably an alpha-keto or 2-oxo acid or lactic acid.

Preferably, the acid comprises an acid selected from the group consisting of 3-methyl-2-oxopentanoic acid, pyruvic acid, 2-oxopentanoic acid, 4-methyl-2-oxopentanoic acid, 3-methyl-2-oxobutanoic acid, 2-oxooctanoic acid, lactic acid and combinations thereof. Advantageously, the acid comprises pyruvic acid or lactic acid. More advantageously, the acid comprises lactic acid.

The liquid storage portion may comprise a liquid storage container containing the liquid aerosol-forming substrate, wherein the hydrochromic material is provided on an internal surface of the liquid storage container. Preferably, at least a portion of the liquid storage container overlying the hydrochromic material is substantially translucent or substantially transparent. Advantageously, a substantially translucent or substantially transparent portion of the liquid storage container may allow a user to observe the colour of the hydrochromic material during use of the aerosol-generating article.

The liquid storage container may be formed from a substantially transparent material, such as ALTUGLAS^{®} Medical Resins Polymethlymethacrylate (PMMA), Chevron Phillips K-Resin^{®} Styrene-butadiene copolymer (SBC), Arkema special performance polymers Pebax^{®}, Rilsan^{®}, and Rilsan^{®} Clear, DOW (Health+^{™}) Low-Density Polyethylene (LDPE), DOW^{™} LDPE 91003, DOW^{™} LDPE 91020 (MFI 2.0; density 923), ExxonMobil^{™} Polypropylene (PP) PP1013H1, PP1014H1 and PP9074MED, Trinseo CALIBRE^{™} Polycarbonate (PC) 2060-SERIES. The liquid storage container may be moulded, such as by in an injection moulding process.

Preferably, the liquid storage container comprises an outlet in the liquid storage container for delivery of the liquid aerosol-forming substrate from the liquid storage container. The outlet may be provided in an end of the liquid storage container. The liquid storage container may comprise a substantially cylindrical container having a closed end and an open end, and a lid comprising the outlet and extending across the open end. The lid may be configured to engage the substantially cylindrical container with an interference fit.

The aerosol-generating article may further comprise a liquid transport element extending through the outlet, the liquid transport element having a first end positioned within the liquid storage container. Advantageously, the liquid transport element may facilitate controlled delivery of the liquid aerosol-forming substrate from the liquid storage container, through the outlet.

The liquid transport element may comprise a capillary wick. The capillary wick may be formed from capillary fibres, including glass fibres, carbon fibres, and metallic fibres, or a combination of any and all of glass fibres, carbon fibres and metallic fibres. Providing metallic fibres may enhance the mechanical resistance of the wick without negatively affecting the hydrophobic properties of the overall wick. Such fibres may be provided parallel to the central axis of the wick, and may be braided, twisted or partially non-woven.

The capillary wick may have a fibrous or spongy structure. The capillary wick preferably comprises a bundle of capillaries. For example, the capillary wick may comprise a plurality of fibres or threads, or other fine bore tubes. The fibres or threads may be generally aligned in a longitudinal direction of the aerosol-generating article. The capillary wick may comprise sponge-like or foam-like material formed into a rod shape. The structure of the wick forms a plurality of small bores or tubes, through which the liquid aerosol-forming substrate can be transported by capillary action. The capillary wick may comprise any suitable material or combination of materials. Examples of suitable materials are ceramic- or graphite-based materials in the form of fibres or sintered powders. The capillary wick may have any suitable capillarity and porosity so as to be used with different liquid physical properties such as density, viscosity, surface tension and vapour pressure. The capillary properties of the wick, combined with the properties of the liquid aerosol-forming substrate, ensure that the wick remains when as long as liquid aerosol-forming substrate remains in the liquid storage container.

The liquid aerosol-forming substrate may comprise a tobacco-containing material containing volatile tobacco flavour compounds which are released from the substrate upon heating. The liquid aerosol-forming substrate may comprise a non-tobacco material. The liquid aerosol-forming substrate may comprise a tobacco-containing material and a non-tobacco containing material. The liquid aerosol-forming substrate may comprise nicotine.

Preferably, the liquid aerosol-forming substrate comprises an aerosol former.

The aerosol-generating article may further comprise an aerosol-generating element configured for aerosolising the liquid aerosol-forming substrate.

In embodiments in which the aerosol-generating article comprises a liquid transport element having a first end positioned within a liquid storage container, the aerosol-generating element may be positioned to aerosolise the liquid aerosol-forming substrate at a second end of the liquid transport element. In use, liquid aerosol-forming substrate is transferred from the liquid storage container towards the aerosol-generating element along the liquid transport element. When the aerosol-generating element is activated, liquid aerosol-forming substrate in the liquid transport element is vaporised by the aerosol-generating element to form a supersaturated vapour. The supersaturated vapour is mixed with and carried in airflow. During the flow, the vapour condenses to form an aerosol and the aerosol is carried towards the mouth of a user.

The aerosol-generating element may comprise a vibratable element, such as a piezoelectric element. The vibratable element may comprise electrical contacts configured to enable an electrical connection to a power supply.

The aerosol-generating element may comprise a susceptor, wherein the susceptor is configured to aerosolise the liquid aerosol-forming substrate when the susceptor is inductively heated.

The aerosol-generating element may comprise an electric heater. The electric heater may comprise electrical contacts configured to enable an electrical connection to a power supply. The electric heater may be a resistive heater. Suitable electrically resistive materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, Constantan, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal^{®}, iron-aluminium based alloys and iron-manganese-aluminium based alloys. Timetal^{®} is a registered trade mark of Titanium Metals Corporation, 1999 Broadway Suite 4300, Denver Colorado. In composite materials, the electrically resistive material may optionally be embedded in, encapsulated or coated with an insulating material or vice-versa, depending on the kinetics of energy transfer and the external physicochemical properties required. The electric heater may comprise a metallic etched foil insulated between two layers of an inert material. In that case, the inert material may comprise Kapton^{®}, all-polyimide or mica foil. Kapton^{®} is a registered trade mark of E.I. du Pont de Nemours and Company, 1007 Market Street, Wilmington, Delaware 19898, United States of America.

The electric heater may comprise an infra-red heating element, a photonic source, or an inductive heating element.

The electric heater may take any suitable form. The electric heater may take the form of a casing or substrate having different electro-conductive portions, or an electrically resistive metallic tube. The electric heater may be a disk (end) heating element or a combination of a disk heating element with heating needles or rods. In embodiments in which the aerosol-generating article comprises a liquid transport element, the electric heater may comprise a flexible sheet of material arranged to surround or partially surround a second end of the liquid transport element. Other possibilities include a heating wire or filament, for example a Ni-Cr, platinum, tungsten or alloy wire, or a heating plate. Optionally, the electric heater may be deposited in or on a rigid carrier material.

According to a second aspect of the present description there is provided an aerosol-generating system comprising an aerosol-generating article according to the first aspect of the present invention, an aerosol-generating element configured for aerosolising the liquid aerosol-forming substrate of the aerosol-generating article, and an aerosol-generating device. The aerosol-generating device comprises an electrical power supply and a controller for controlling a supply of electrical power from the electrical power supply to the aerosol-generating element. The aerosol-generating system is configured so that the hydrochromic material of the aerosol-generating article is visible from the exterior of the aerosol-generating system.

In aerosol-generating systems according to the second aspect , the hydrochromic material is visible from the exterior of the aerosol-generating system. Therefore, advantageously, a user can observe the hydrochromic material during use of the aerosol-generating system to determine when the liquid aerosol-forming substrate has been exhausted.

Preferably, at least one of the aerosol-generating article and the aerosol-generating device comprises a translucent portion or a transparent portion overlying the hydrochromic material. Preferably, the aerosol-generating article comprises a translucent portion or a transparent portion overlying the hydrochromic material.

The aerosol-generating device may comprise a housing defining a cavity for receiving at least part of the aerosol-generating article. In such embodiments, the housing may comprise a translucent portion or a transparent portion configured to overlie the hydrochromic material when at least a portion of the aerosol-generating article is received within the cavity.

The aerosol-generating element may form part of the aerosol-generating article, as described herein with reference to the first aspect of the present invention. The aerosol-generating device may comprise electrical contacts configured to electrically connect with electrical contacts on the aerosol-generating element.

The aerosol-generating element may form part of the aerosol-generating device.

The aerosol-generating element may be provided separately from both the aerosol-generating article and the aerosol-generating device, wherein the aerosol-generating element is combined with the aerosol-generating article and the aerosol-generating device to form the aerosol-generating system. In embodiments in which the aerosol-generating element is configured for use with multiple aerosol-generating articles, an aerosol-generating element that is provided separately from both the aerosol-generating article and the aerosol-generating device may be advantageous. For example, an aerosol-generating element that is provided separately from the aerosol-generating article and the aerosol-generating device may facilitate cleaning of the aerosol-generating element. The aerosol-generating device may comprise electrical contacts configured to electrically connect with electrical contacts on the aerosol-generating element.

Suitable aerosol-generating elements are described herein with reference to the first aspect of the present invention.

The electrical power supply may comprise a direct current (DC) source. In preferred embodiments, the electrical power supply comprises a battery. The electrical power supply may comprise a Nickel-metal hydride battery, a Nickel cadmium battery, or a Lithium based battery, for example a Lithium-Cobalt, a Lithium-Iron-Phosphate or a Lithium-Polymer battery.

The aerosol-generating device may further comprise an electronic photosensor configured to sense an optical property of the hydrochromic material of the aerosol-generating article when the aerosol-generating article is combined with the aerosol-generating device. The controller is configured to monitor a value of the sensed optical property when the aerosol-generating device is operated in combination with the aerosol-generating article. The controller is configured to control a supply of electrical power from the electrical power supply to the aerosol-generating element when the value of the sensed optical property is within a first range indicative of the first colour. The controller is configured to prevent a supply of electrical power from the electrical power supply to the aerosol-generating element when the value of the sensed optical property is outside the first range and indicative of the second colour. This may be particularly advantageous in embodiments in which the aerosol-generating element comprises an electric heater, since the controller is configured to prevent further heating of the aerosol-generating article when the second colour is detected. That is, the controller is configured to prevent further heating when the liquid aerosol-forming substrate has been exhausted.

The controller may be configured to repeatedly measure the value of the sensed optical property during operation of the aerosol-generating device in combination with the aerosol-generating article to determine when the value of the sensed optical property no longer falls within the first range. The controller may be configured to periodically measure the value of the sensed optical property. The controller may be configured to continuously measure the value of the sensed optical property during operation of the aerosol-generating device in combination with the aerosol-generating article.

The controller may be configured to estimate the amount of liquid aerosol-forming substrate remaining in the aerosol-generating article based on the measured value of the sensed optical property of the hydrochromic material. As described herein with reference to the first aspect of the present description, the hydrochromic material may gradually change from the first colour to the second colour as the liquid aerosol-forming substrate is consumed. The aerosol-generating device may comprise a feedback device for providing feedback to the user indicative of the estimated amount of liquid aerosol-forming substrate remaining.

The optical property may comprise at least one of reflectance, absorbance, transmittance, colour, and combinations thereof.

According to a third aspect of the present description, and according to the present invention, there is provided an aerosol-generating device configured for combination with an aerosol-generating article. Preferably the aerosol-generating device is configured for combination with an aerosol-generating article according to the first aspect of the present invention. The aerosol-generating device comprises an electrical power supply, an electronic photosensor and a controller. The electronic photosensor is configured to sense an optical property of a portion of an aerosol-generating article when the aerosol-generating article is combined with the aerosol-generating device. The controller is configured to monitor a value of the sensed optical property when the aerosol-generating device is operated in combination with an aerosol-generating article. The controller is configured to control a supply of electrical power from the electrical power supply to an aerosol-generating element when the value of the sensed optical property is within a first range. The controller is configured to prevent a supply of electrical power from the electrical power supply to the aerosol-generating element when the value of the sensed optical property is outside the first range. The first range may comprise any value above or below a predetermined threshold value.

The controller is configured to repeatedly measure the value of the sensed optical property during operation of the aerosol-generating device in combination with an aerosol-generating article to determine when the value of the sensed optical property no longer falls within the first range. The controller may be configured to periodically measure the value of the sensed optical property. The controller may be configured to continuously measure the value of the sensed optical property during operation of the aerosol-generating device in combination with an aerosol-generating article.

The controller may be configured to estimate the amount of a liquid aerosol-forming substrate remaining in the aerosol-generating article based on the measured value of the sensed optical property. In embodiments in which the aerosol-generating device is configured for use with an aerosol-generating article according to the first aspect of the present invention, the controller may be configured to estimate the amount of liquid aerosol-forming substrate remaining in the liquid storage portion based on a measured value of a sensed optical property of the hydrochromic material. The hydrochromic material may gradually change from the first colour to the second colour as the liquid aerosol-forming substrate is consumed. The aerosol-generating device may comprise a feedback device for providing feedback to the user indicative of the estimated amount of liquid aerosol-forming substrate remaining.

In embodiments in which the controller is configured for combination with an aerosol-generating article according to the first aspect of the present invention, values of the sensed optical property within the first range may be indicative of the first colour of the hydrochromic material. Values of the sensed optical property outside the first range may be indicative of the second colour of the hydrochromic material.

The optical property may comprise at least one of reflectance, absorbance, transmittance, colour, and combinations thereof.

The aerosol-generating device is configured for combination with an aerosol-generating article. That is, the aerosol-generating device is configured to be operatively connected with an aerosol-generating article. The aerosol-generating device may comprise a cavity for receiving at least a portion of an aerosol-generating article. The aerosol-generating device may comprise an attachment portion for releasably attaching the aerosol-generating device to an aerosol-generating article. The attachment portion may comprise a screw thread for engaging a corresponding screw thread on an aerosol-generating article. The attachment portion may be configured to engage a corresponding attachment portion on an aerosol-generating article by an interference fit.

The aerosol-generating device may further comprise an aerosol-generating element. Suitable aerosol-generating elements are described herein with reference to the first aspect of the present invention.

The aerosol-generating device may be configured for combination with an aerosol-generating article comprising an aerosol-generating element. The aerosol-generating device may be configured for combination with an aerosol-generating article and a separate aerosol-generating element. The aerosol-generating device may comprise electrical contacts configured to electrically connect with electrical contacts on an aerosol-generating element.

The aerosol-generating device may comprise any of the optional or preferred features described herein with reference to aerosol-generating devices forming part of the aerosol-generating system according to the second aspect of the present description.

The aerosol-generating device according to the third aspect , i.e. the present invention may be combined with the aerosol-generating article according to the first aspect of the present description to form an aerosol-generating system.

The invention is further described, by way of example only, with reference to the accompanying drawings in which:
Figure 1 shows an aerosol-generating article according to a first embodiment of the present description;
Figure 2 shows an aerosol-generating device for use with the aerosol-generating article of Figure 1;
Figure 3 shows the aerosol-generating article of Figure 1 after partial use; and
Figure 4 shows an aerosol-generating system according to a second embodiment of the present description;

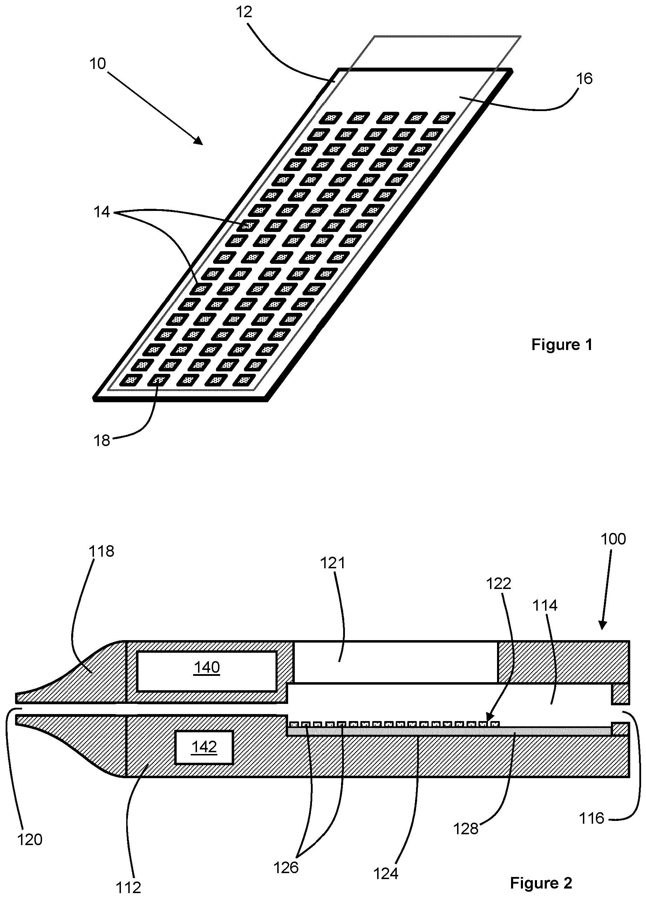

Figure 1 shows an aerosol-generating article 10 according to a first aspect of the description.

The aerosol-generating article 10 comprises a base layer 12 and a plurality of discrete liquid storage portions 14 positioned on the base layer 12. A removable cover layer 16 is secured to the base layer 12 so that the plurality of liquid storage portions 14 is sealed between the base layer 12 and the cover layer 16.

Each of the liquid storage portions 14 comprises a porous substrate material and a liquid aerosol-forming substrate sorbed onto the porous substrate material. A hydrochromic material 18 is provided on a surface of each of the porous substrate materials. The hydrochromic material 18 is configured to be substantially transparent when in contact with the liquid aerosol-forming substrate so that the colour of the underlying porous substrate material is visible.

Figure 2 shows a cross-sectional view of an aerosol-generating device 100 for use with the aerosol-generating article 10 of Figure 1. The aerosol-generating device 100 comprises a housing 112 defining a cavity 114 for receiving the aerosol-generating article 10. An air inlet 116 is provided at an upstream end of the cavity 114 and a mouthpiece 118 is provided at a downstream end of the housing 112. An air outlet 120 is provided in the mouthpiece 118 in fluid communication with the cavity 114 so that an airflow path is defined through the cavity 114 between the air inlet 116 and the air outlet 120. During use, a user draws on the mouthpiece 118 to draw air into the cavity 114 through the air inlet 116 and out of the cavity 114 through the air outlet 20.

A transparent window 121 provided in the housing 112 allows a user to observe the aerosol-generating article 10 when the aerosol-generating article 10 is received within the cavity 114.

The aerosol-generating device 100 further comprises a plurality of aerosol-generating elements 122 provided on a planar wall 124 of the cavity 114. Each of the aerosol-generating elements 122 comprises an electric heater element 126 provided on a common support layer 128.

The aerosol-generating device 100 further comprises an electrical power supply 140 and a controller 142 positioned within the housing 112. During operation of the aerosol-generating device 100, the controller 142 controls a supply of electrical current from the electrical power supply 140 to each aerosol-generating element 122 to activate the aerosol-generating element 122. The controller 142 may be configured to activate the plurality of aerosol-generating elements 122 in groups, with each group being activated and deactivated sequentially.

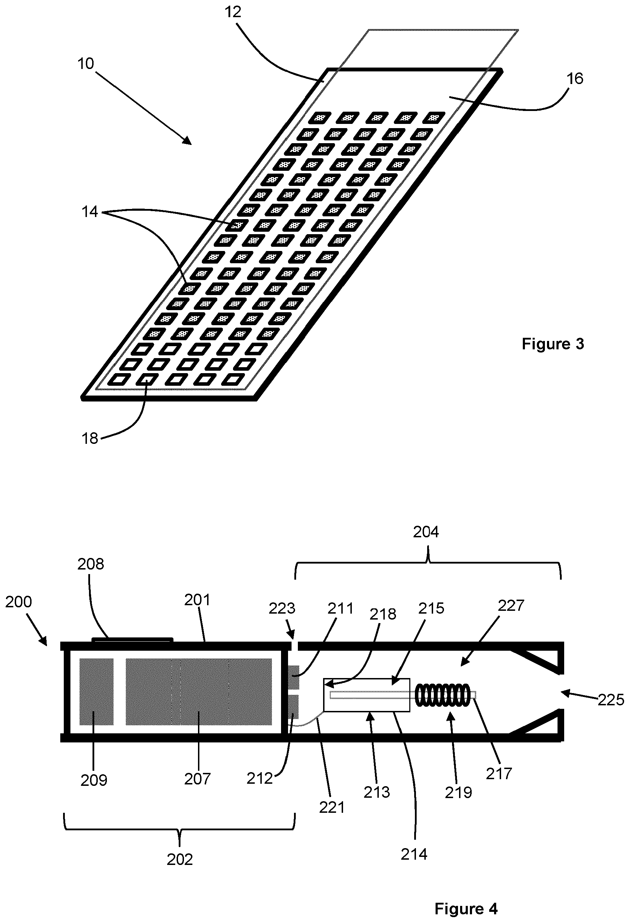

During use, the aerosol-generating article 10 is inserted into the cavity 114 so that the aerosol-generating article 10 and the aerosol-generating device 100 form an aerosol-generating system. The controller 142 then sequentially activates and deactivates the aerosol-generating elements 122 to sequentially heat the discrete liquid storage portions 14. Each time a liquid storage portion 14 is heated the liquid aerosol-forming substrate is aerosolised until substantially no liquid aerosol-forming substrate remains in the porous substrate material. In the absence of the liquid aerosol-forming substrate, the hydrochromic material 18 on the porous substrate material changes from being substantially transparent to an opaque colour, such as white. Figure 3 shows the aerosol-generating article 10 of Figure 1 after some of the liquid storage portions 14 have been heated and the hydrochromic material 18 has been transformed from substantially transparent to white.

During use, the user may observe the aerosol-generating article 10 through the transparent window 121 of the aerosol-generating device 100 to inspect the colour of the hydrochromic material 18 on each liquid storage portion 14. In this way, the user can determine how many of the liquid storage portions 14 have been heated.

Figure 4 shows an aerosol-generating system 200 according to a second aspect of the present description. The aerosol-generating system 200 comprises an aerosol-generating device 202 and an aerosol-generating article 204 removably attached to the aerosol-generating device. The aerosol-generating system may be an electronic smoking system in which the aerosol-generating device 202 is a main body of the electronic smoking system and the aerosol-generating article 204 is a replaceable cartridge, such as a cartomiser.

The aerosol-generating device 202 comprises a housing 201, an electrical power supply 207, a feedback device 208, a controller 209, a puff detection system 211 and an electronic photosensor 212.

The aerosol-generating article 204 comprises a liquid storage portion 213 comprising a transparent liquid storage container 214 containing a liquid aerosol-forming substrate 215. The aerosol-generating article 204 further comprises a liquid transport element 217 in the form of a capillary wick, and an aerosol-generating element 219 comprising an electric heater. A first end of the capillary wick extends into the liquid storage container 214 and a second end of the capillary wick is surrounded by the electric heater. The electric heater is connected to the aerosol-generating device 202 via electrical connections 221.

A hydrochromic material 218 is provided on an internal surface of the liquid storage container 214, in contact with the liquid aerosol-forming substrate 215. The hydrochromic material 218 is configured to exhibit a first colour when in contact with the liquid aerosol-forming substrate and a second colour when the liquid aerosol-forming substrate 215 has been exhausted from the liquid storage container 214. The first colour may be transparent and the second colour may be opaque.

The aerosol-generating article 204 also includes an air inlet 223, an air outlet 225 and an aerosol-forming chamber 227.

During use, liquid aerosol-forming substrate 215 is transferred or conveyed by capillary action from the liquid storage container 214 from the first end of the wick 217 to the second end of the wick 217, which is surrounded by the electric heater. When a user draws on the device at the air outlet 225, ambient air is drawn through air inlet 223. The puff detection system 211 senses the puff and activates the electric heater. The electrical power supply 207 supplies energy to the electric heater to heat the end of the wick 217 surrounded by the electric heater. The liquid aerosol-forming substrate 215 in the second end of the wick 217 is vaporised by the electric heater to create a supersaturated vapour. At the same time, the liquid aerosol-forming substrate 215 being vaporised is replaced by further liquid aerosol-forming substrate 215 moving along the wick 217 by capillary action. The supersaturated vapour created is mixed with and carried in the airflow from the air inlet 223. In the aerosol-forming chamber 227, the vapour condenses to form an inhalable aerosol, which is carried towards the outlet 225 and into the mouth of the user.

During operation of the aerosol-generating system 200, the electronic photosensor 212 senses an optical property of the hydrochromic material 218 through the transparent liquid storage container 214. As the liquid aerosol-forming substrate 215 is consumed from the liquid storage container 215 the hydrochromic material 218 gradually changes from the first colour to the second colour. The controller 209 monitors the value of the sensed optical property and continuously estimates the amount of liquid aerosol-forming substrate 215 remaining in the liquid storage container 214. The estimated amount of liquid aerosol-forming substrate 215 remaining is displayed on the feedback device 208. When the value of the sensed optical property of the hydrochromic material 218 is indicative of the second colour the controller 209 prevents further activation of the electric heater. The present invention is set out in the appended claims. The embodiments, examples or aspects according to the present description which do not fall within the scope of said claims are provided for illustrative purposes only and do not form part of the present invention.

## Claims

1. An aerosol-generating device (202) configured for combination with an aerosol-generating article (204), the aerosol-generating device (202) comprising:
an electrical power supply (207);
an electronic photosensor (212) configured to sense an optical property of a portion of an aerosol-generating article (204) when the aerosol-generating article (204) is combined with the aerosol-generating device (202); and
a controller (209) configured to repeatedly measure a value of the sensed optical property when the aerosol-generating device (202) is operated in combination with an aerosol-generating article (204), the controller (209) configured to control a supply of electrical power from the electrical power supply (207) to an aerosol-generating element (219) when the value of the sensed optical property is within a first range, wherein the controller (209) is configured to prevent a supply of electrical power from the electrical power supply (207) to the aerosol-generating element (219) when the value of the sensed optical property is outside the first range.

2. An aerosol-generating device (202) according to claim 1, wherein the controller (209) is configured to periodically measure the value of the sensed optical property when the aerosol-generating device (202) is operated in combination with the aerosol-generating article (204).

3. An aerosol-generating device (202) according to claim 1, wherein the controller (209) is configured to continuously measure the value of the sensed optical property when the aerosol-generating device (202) is operated in combination with the aerosol-generating article (204).

4. An aerosol-generating device (202) according to claim 1, 2 or 3, wherein the optical property comprises at least one of reflectance, absorbance, transmittance, and colour.

5. An aerosol-generating device (202) according to any preceding claim, wherein the controller (209) is configured to estimate the amount of a liquid aerosol-forming substrate (215) remaining in the aerosol-generating article (204) based on the measured value of the sensed optical property.

6. An aerosol-generating device (202) according to claim 5, further comprising a feedback device (208) for providing feedback to a user indicative of the estimated amount of liquid aerosol-forming substrate (215) remaining in the aerosol-generating article (204).

7. An aerosol-generating system (200) comprising an aerosol-generating device (202) according to any preceding claim and an aerosol-generating article (204), the aerosol-generating article (204) comprising:
a liquid storage portion (213) containing a liquid aerosol-forming substrate (215); and
a hydrochromic material (218) provided on the liquid storage portion (213), wherein the hydrochromic material (218) has a first colour when in contact with the liquid aerosol-forming substrate (215) and a second colour in the absence of the liquid aerosol-forming substrate (215).

8. An aerosol-generating system (200) according to claim 7, wherein the values of the sensed optical property within the first range are indicative of the first colour of the hydrochromic material (218), and wherein values of the sensed optical property outside the first range are indicative of the second colour of the hydrochromic material (218).

9. An aerosol-generating system (200) according to claim 7 or 8, wherein the aerosol-generating article further comprises a base layer, wherein the liquid storage portion comprises a porous substrate material positioned on the base layer and the liquid aerosol-forming substrate sorbed into the porous substrate material.

10. An aerosol-generating system (200) according to claim 9, wherein the hydrochromic material is provided on an outer surface of the porous substrate material.

11. An aerosol-generating system (200) according to claim 7 or 8, wherein the liquid storage portion (213) comprises a liquid storage container (214) containing the liquid aerosol-forming substrate (215), and wherein the hydrochromic material (218) is provided on an internal surface of the liquid storage container (214).

12. An aerosol-generating system (200) according to claim 11, further comprising an outlet in the liquid storage container (214) for delivery of the liquid aerosol-forming substrate (215) from the liquid storage container (214).

13. An aerosol-generating system (200) according to claim 12, further comprising a liquid transport element (217) extending through the outlet, the liquid transport element (217) having a first end positioned within the liquid storage container (214).

14. An aerosol-generating system (200) according to any of claims 7 to 13, wherein the liquid aerosol-forming substrate (215) comprises water.

15. An aerosol-generating system (200) according to any of claims 7 to 14, wherein the liquid aerosol-forming substrate (215) comprises nicotine.

## Patentansprüche

1. Aerosolerzeugungsvorrichtung (202), ausgelegt zu einer Kombination mit einem aerosolerzeugenden Artikel (204), die Aerosolerzeugungsvorrichtung (202) aufweisend:
eine elektrische Energieversorgung (207);
einen elektronischen Fotosensor (212), der zur Erfassung einer optischen Eigenschaft eines Abschnitts eines aerosolerzeugenden Artikels (204) ausgelegt ist, wenn der aerosolerzeugende Artikel (204) mit der Aerosolerzeugungsvorrichtung (202) kombiniert wird; und
einen Regler (209), der zur wiederholten Messung eines Werts der erfassten optischen Eigenschaft ausgelegt ist, wenn die Aerosolerzeugungsvorrichtung (202) in Kombination mit einem aerosolerzeugenden Artikel (204) in Betrieb ist, wobei der Regler (209) zur Regelung einer Zufuhr von elektrischer Energie von der elektrischen Energieversorgung (207) an ein aerosolerzeugendes Element (219) ausgelegt ist, wenn der Wert der erfassten optischen Eigenschaft innerhalb eines ersten Bereichs liegt, wobei der Regler (209) zur Verhinderung einer Zufuhr von elektrischer Energie von der elektrischen Energieversorgung (207) an das aerosolerzeugende Element (219) ausgelegt ist, wenn der Wert der erfassten optischen Eigenschaft außerhalb des ersten Bereichs liegt.

2. Aerosolerzeugungsvorrichtung (202) nach Anspruch 1, wobei der Regler (209) zur regelmäßigen Messung des Werts der erfassten optischen Eigenschaft ausgelegt ist, wenn die Aerosolerzeugungsvorrichtung (202) in Kombination mit dem aerosolerzeugenden Artikel (204) in Betrieb ist.

3. Aerosolerzeugungsvorrichtung (202) nach Anspruch 1, wobei der Regler (209) zur kontinuierlichen Messung des Werts der erfassten optischen Eigenschaft ausgelegt ist, wenn die Aerosolerzeugungsvorrichtung (202) in Kombination mit dem aerosolerzeugenden Artikel (204) in Betrieb ist.

4. Aerosolerzeugungsvorrichtung (202) nach Anspruch 1, 2 oder 3, wobei die optische Eigenschaft zumindest eines von Reflexionsvermögen, Absorptionsvermögen, Transmissionsvermögen und Farbe aufweist.

5. Aerosolerzeugungsvorrichtung (202) nach einem der vorhergehenden Ansprüche, wobei der Regler (209) zum Schätzen der Menge eines in dem aerosolerzeugenden Artikel (204) zurückbleibenden, flüssigen aerosolbildenden Substrats (215), basierend auf dem gemessenen Wert der erfassten optischen Eigenschaft, ausgelegt ist.

6. Aerosolerzeugungsvorrichtung (202) nach Anspruch 5, ferner aufweisend eine Rückmeldevorrichtung (208) zum Vorsehen einer Rückmeldung an einen Benutzer, das die geschätzte Menge des in dem aerosolerzeugenden Artikel (204) zurückbleibenden, flüssigen aerosolbildenden Substrats (215) anzeigt.

7. Aerosolerzeugungssystem (200), aufweisend eine Aerosolerzeugungsvorrichtung (202) nach einem der vorhergehenden Ansprüche und einen aerosolerzeugenden Artikel (204), der aerosolerzeugende Artikel (204) aufweisend:
einen Flüssigspeicherteil (213), ein flüssiges aerosolbildendes Substrat (215) enthaltend; und
ein hydrochromes Material (218), das in dem Flüssigspeicherteil (213) vorgesehen ist, wobei das hydrochrome Material (218) eine erste Farbe hat, wenn es mit dem flüssigen aerosolbildenden Substrat (215) in Kontakt ist, und eine zweite Farbe hat, wenn das flüssige aerosolbildende Substrat (215) nicht vorhanden ist.

8. Aerosolerzeugungssystem (200) nach Anspruch 7, wobei die Werte der erfassten optischen Eigenschaft innerhalb des ersten Bereichs die erste Farbe des hydrochromen Materials (218) anzeigen und wobei Werte der erfassten optischen Eigenschaft außerhalb des ersten Bereichs die zweite Farbe des hydrochromen Materials (218) anzeigen.

9. Aerosolerzeugungssystem (200) nach Anspruch 7 oder 8, wobei der aerosolerzeugende Artikel ferner eine Basisschicht aufweist, wobei der Flüssigspeicherteil ein poröses auf der Basisschicht positioniertes Substratmaterial aufweist und das flüssige aerosolbildende Substrat in das poröse Substratmaterial sorbiert ist.

10. Aerosolerzeugungssystem (200) nach Anspruch 9, wobei das hydrochrome Material auf einer Außenfläche des porösen Substratmaterials vorgesehen ist.

11. Aerosolerzeugungssystem (200) nach Anspruch 7 oder 8, wobei der Flüssigspeicherteil (213) einen das flüssige aerosolbildende Substrat (215) enthaltenden Behälter zur Flüssigkeitsspeicherung (214) aufweist, und wobei das hydrochrome Material (218) auf einer Innenfläche des Behälters zur Flüssigkeitsspeicherung (214) vorgesehen ist.

12. Aerosolerzeugungssystem (200) nach Anspruch 11, ferner aufweisend einen Auslass in dem Behälter zur Flüssigkeitsspeicherung (214) zur Abgabe des flüssigen aerosolbildenden Substrats (215) aus dem Behälter zur Flüssigkeitsspeicherung (214).

13. Aerosolerzeugungssystem (200) nach Anspruch 12, ferner aufweisend ein sich durch den Auslass erstreckendes Flüssigkeitstransportelement (217), wobei das Flüssigkeitstransportelement (217) ein innerhalb des Behälters zur Flüssigkeitsspeicherung (214) positioniertes erstes Ende hat.

14. Aerosolerzeugungssystem (200) nach einem der Ansprüche 7 bis 13, wobei das flüssige aerosolbildende Substrat (215) Wasser aufweist.

15. Aerosolerzeugungssystem (200) nach einem der Ansprüche 7 bis 14, wobei das flüssige aerosolbildende Substrat (215) Nikotin aufweist.

## Revendications

1. Dispositif de génération d'aérosol (202) configuré pour une combinaison avec un article de génération d'aérosol (204), le dispositif de génération d'aérosol (202) comprenant :
une alimentation électrique (207) ;
un photocapteur électronique (212) configuré pour détecter une propriété optique d'une partie d'un article de génération d'aérosol (204) lorsque l'article de génération d'aérosol (204) est combiné avec le dispositif de génération d'aérosol (202) ; et
un dispositif de commande (209) configuré pour mesurer de manière répétée une valeur de la propriété optique détectée lorsque le dispositif de génération d'aérosol (202) fonctionne en combinaison avec un article de génération d'aérosol (204), le dispositif de commande (209) étant configuré pour commander une alimentation en énergie électrique depuis l'alimentation électrique (207) jusqu'à un élément de génération d'aérosol (219) lorsque la valeur de la propriété optique détectée est dans une première plage, dans lequel le dispositif de commande (209) est configuré pour empêcher une alimentation en énergie électrique depuis l'alimentation électrique (207) jusqu'à l'élément de génération d'aérosol (219) lorsque la valeur de la propriété optique détectée est en dehors de la première plage.

2. Dispositif de génération d'aérosol (202) selon la revendication 1, dans lequel le dispositif de commande (209) est configuré pour mesurer périodiquement la valeur de la propriété optique détectée lorsque le dispositif de génération d'aérosol (202) est mis en fonctionnement en combinaison avec l'article de génération d'aérosol (204).

3. Dispositif de génération d'aérosol (202) selon la revendication 1, dans lequel le dispositif de commande (209) est configuré pour mesurer continuellement la valeur de la propriété optique détectée lorsque le dispositif de génération d'aérosol (202) est mis en fonctionnement en combinaison avec l'article de génération d'aérosol (204).

4. Dispositif de génération d'aérosol (202) selon la revendication 1, 2 ou 3, dans lequel la propriété optique comprend au moins l'une parmi une réflectance, une absorbance, une transmittance et une couleur.

5. Dispositif de génération d'aérosol (202) selon l'une quelconque des revendications précédentes, dans lequel le dispositif de commande (209) est configuré pour estimer la quantité d'un substrat formant aérosol liquide (215) restant dans l'article de génération d'aérosol (204) sur la base de la valeur mesurée de la propriété optique détectée.

6. Dispositif de génération d'aérosol (202) selon la revendication 5, comprenant en outre un dispositif de rétroaction (208) destiné à fournir une rétroaction à un utilisateur indicative de la quantité estimée de substrat formant aérosol liquide (215) restant dans l'article de génération d'aérosol (204).

7. Système de génération d'aérosol (200) comprenant un dispositif de génération d'aérosol (202) selon l'une quelconque des revendications précédentes et un article de génération d'aérosol (204), l'article de génération d'aérosol (204) comprenant :
une partie de stockage de liquide (213) contenant un substrat formant aérosol liquide (215) ; et
un matériau hydrochromique (218) fourni sur la partie de stockage de liquide (213), dans lequel le matériau hydrochromique (218) a une première couleur lorsqu'il est en contact avec le substrat formant aérosol liquide (215) et une deuxième couleur en l'absence du substrat formant aérosol liquide (215).

8. Système de génération d'aérosol (200) selon la revendication 7, dans lequel les valeurs de la propriété optique détectée à l'intérieur de la première plage sont indicatives de la première couleur du matériau hydrochromique (218), et dans lequel les valeurs de la propriété optique détectée en dehors de la première plage sont indicatives de la deuxième couleur du matériau hydrochrome (218).

9. Système de génération d'aérosol (200) selon la revendication 7 ou 8, dans lequel l'article de génération d'aérosol comprend en outre une couche de base, dans lequel la partie de stockage de liquide comprend un matériau de substrat poreux positionné sur la couche de base et le substrat formant aérosol liquide sorbé dans le matériau de substrat poreux.

10. Système de génération d'aérosol (200) selon la revendication 9, dans lequel le matériau hydrochromique est fourni sur une surface extérieure du matériau de substrat poreux.

11. Système de génération d'aérosol (200) selon la revendication 7 ou 8, dans lequel la partie de stockage de liquide (213) comprend un récipient de stockage de liquide (214) contenant le substrat formant aérosol liquide (215), et dans lequel le matériau hydrochromique (218) est fourni sur une surface intérieure du récipient de stockage de liquide (214).

12. Système de génération d'aérosol (200) selon la revendication 11, comprenant en outre une sortie dans le récipient de stockage de liquide (214) destiné à libérer le substrat formant aérosol liquide (215) du récipient de stockage de liquide (214).

13. Système de génération d'aérosol (200) selon la revendication 12, comprenant en outre un élément de transport de liquide (217) s'étendant à travers la sortie, l'élément de transport de liquide (217) ayant une première extrémité positionnée à l'intérieur du récipient de stockage de liquide (214).

14. Système de génération d'aérosol (200) selon l'une quelconque des revendications 7 à 13, dans lequel le substrat formant aérosol liquide (215) comprend de l'eau.

15. Système de génération d'aérosol (200) selon l'une quelconque des revendications 7 à 14, dans lequel le substrat formant aérosol liquide (215) comprend de la nicotine.
